(19) Europäisches Patentamt
European Patent Office
Office européen des brevets

(11) Veröffentlichungsnummer: **0 149 976**
**A2**

(12) # EUROPÄISCHE PATENTANMELDUNG

(21) Anmeldenummer: **84810643.1**

(22) Anmeldetag: **19.12.84**

(51) Int. Cl.⁴: **C 07 D 233/56**
C 07 D 233/60, A 61 K 31/415

(30) Priorität: **30.12.83 CH 6988/83**

(43) Veröffentlichungstag der Anmeldung:
**31.07.85 Patentblatt 85/31**

(84) Benannte Vertragsstaaten:
**AT BE CH DE FR GB IT LI LU NL SE**

(71) Anmelder: **CIBA-GEIGY AG**
**Postfach**
**CH-4002 Basel(CH)**

(72) Erfinder: **Züst, Armin, Dr.**
**Sternengasse 27/408**
**CH-4051 Basel(CH)**

(72) Erfinder: **Schilling, Walter, Dr.**
**Im Muspenacker**
**CH-4249 Himmelried(CH)**

(72) Erfinder: **Paioni, Romeo, Dr.**
**Wettsteinstrasse 6**
**CH-4125 Riehen(CH)**

(54) **Substituierte Imidazole.**

(57) Substituierte Imidazole der allgemeinen Formel

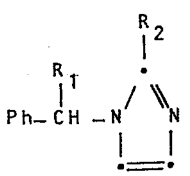

(I)

oder deren Salze, worin Ph unsubstituiertes oder durch Niederalkyl oder Niederalkoxy substituiertes Phenyl bedeutet und $R_1$ und $R_2$ jeweils Niederalkyl bedeuten, Verfahren zu ihrer Herstellung, ihre Verwendung sowie pharmazeutische Präparate.

Die Verbindungen der Formel I weisen beispielseise antidepressive Eigenschaften auf.

EP 0 149 976 A2

Croydon Printing Company Ltd.

0149976



CIBA-GEIGY AG
Basel (Schweiz)

4-14722/+

## Substituierte Imidazole

Die Erfindung betrifft substituierte Imidazole der allgemeinen Formel

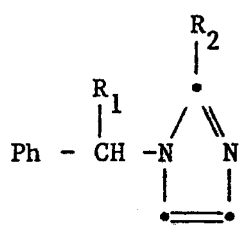

(I)

oder deren Salze, worin Ph unsubstituiertes oder durch Niederalkyl oder Niederalkoxy substituiertes Phenyl bedeutet und $R_1$ und $R_2$ jeweils Niederalkyl bedeuten, ihre Verwendung, Verfahren zu ihrer Herstellung und pharmazeutische Präparate, enthaltend eine Verbindung der Formel I oder ein pharmazeutisch verwendbares Salz davon.

Durch Niederalkyl oder Niederalkoxy substituiertes Phenyl weist insbesondere ein oder mehrere, z.B. zwei, ferner drei, in erster Linie in ortho- und/oder meta-Position gebundene, Niederalkyl- oder Niederalkoxyreste auf.

Vor- und nachstehend sind unter mit "nieder" bezeichneten Resten oder Verbindungen vorzugsweise solche zu verstehen, die bis und mit 7, vor allem bis und mit 4 Kohlenstoffatome, enthalten.

Die verwendeten Allgemeinbegriffe haben folgende Bedeutungen:

Niederalkyl ist z.B. Methyl, Ethyl, n-Propyl, Isopropyl, n-Butyl, Isobutyl, sek-Butyl, tert.-Butyl und umfasst ferner entsprechende Pentyl-, Hexyl- oder Heptylreste.

Niederalkoxy ist z.B. Methoxy, Ethoxy, n-Propyloxy, Isopropyloxy, Pentyloxy, Isobutyloxy und tert-Butyloxy.

Salze von Verbindungen der Formel I sind deren Säureadditionssalze, vorzugsweise pharmazeutisch verwendbare Säureadditionssalze. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie Niederalkancarbonsäuen, z.B. Essigsäure, wie gegebenenfalls ungesättigten Dicarbonsäuren, z.B. Malon-, Malein- oder Fumarsäure, oder wie Hydroxycarbonsäuren, z.B. Wein-oder Citronensäure, oder mit Sulfonsäuren, wie Niederalkan- oder gegebenenfalls substituierten Benzolsulfonsäuren, z.B. Methan- oder p-Toluol-sulfonsäure, gebildet. Umfasst sind ferner für pharmazeutische Verwendungen ungeeignete Salze, da diese beispielsweise für die Isolierung bzw. Reinigung freier erfindungsgemässer Verbindungen sowie derer pharmazeutisch verwendbarer Salze verwendet werden können.

Die erfindungsgemässen Verbindungen weisen z.B. wertvolle pharmakologische Eigenschaften auf. Insbesondere besitzen sie die Fähigkeit, die Monoaminooxidase (MAO) vom Typ A selektiv und reversibel zu hemmen. Diese Eigenschaften können sowohl in der Rattenleber als auch im Rattenhirn nachgewiesen werden, wo jeweils der Abbau des Serotonins selektiv gehemmt wird. Die Bestimmung der MAO-Aktivität in der Rattenleber bzw. im Rattenhirn wurde analog der Methodik von R.J. Wurtman et al., Biochem. Pharmacol. 12, 1439 (1963) durchgeführt, wobei die MAO-Hemmung ab einer Dosis von etwa 1 mg/kg nach peroraler Applikation des Wirkstoffs festzustellen ist.

Entsprechend können die erfindungsgemässen Verbindungen zur prophylaktischen und therapeutischen Behandlung depressiver Zustände verwendet werden.

Ein weiterer Gegenstand der Erfindung ist somit die prophylaktische und therapeutische Behandlung des menschlichen, ferner des tierischen Körpers sowie die Verwendung der erfindungsgemässen Verbindungen zur Behandlung depressiver Zustände.

Die Erfindung betrifft beispielsweise Verbindungen der Formel I oder deren Salze, worin Ph unsubstituiertes oder ein- oder mehrfach, z.B. zweifach, durch Niederalkyl, insbesondere mit bis und mit 4 C-Atomen, substituiertes Phenyl bedeutet und $R_1$ und $R_2$ jeweils Niederalkyl, insbesondere mit bis und mit 4 C-Atomen, bedeuten.

Die Erfindung betrifft beispielsweise Verbindungen der Formel I oder deren Salze, worin Ph ein- oder mehrfach, z.B. zweifach, durch Niederalkoxy, insbesondere mit bis und mit 4 C-Atomen, wie Methoxy, substituiertes Phenyl bedeutet, und $R_1$ und $R_2$ jeweils Niederalkyl, insbesondere mit bis und mit 4 C-Atomen, wie Methyl, bedeuten.

Die Erfindung betrifft insbesondere Verbindungen der Formel I oder deren Salze, worin Ph unsubstituiertes oder in Position 2 oder 3 einfach durch Niederalkyl oder Niederalkoxy jeweils mit bis und mit 4 C-Atomen, wie Methyl oder Methoxy, oder in Position 2 und 6 zweifach durch Niederalkyl mit bis und mit 4 C-Atomen, wie Methyl, substituiertes Phenyl bedeutet und $R_1$ und $R_2$ jeweils Niederalkyl mit bis und mit 4 C-Atomen, wie Methyl, bedeuten.

Die Erfindung betrifft insbesondere Verbindungen der Formel I oder deren Salze, worin Ph unsubstituiertes Phenyl bedeutet und $R_1$ und $R_2$ jeweils Niederalkyl mit bis und mit 4 C-Atomen, wie Methyl, bedeuten.

Die Erfindung betrifft namentlich die in den Beispielen genannten neuen Verbindungen und ihre Salze.

Ein weiterer Erfindungsgegenstand ist ein Verfahren zur Herstellung der Verbindungen der Formel I und ihrer Salze, dadurch gekennzeichnet, dass man

a) eine Verbindung der Formel

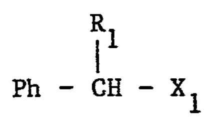

(IIa),

worin $X_1$ reaktionsfähiges verestertes Hydroxy bedeutet, mit einer Verbindung der Formel

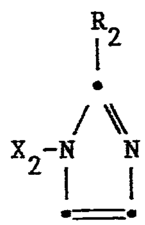

(IIb),

worin $X_2$ für Wasserstoff oder einen metallischen Rest steht, umsetzt, oder

b) in einer Verbindung der Formel

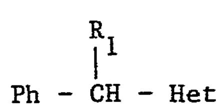

(III),

worin Het für einen in 2-$R_2$-Imidazol-1-yl überführbaren Rest steht, Het in 2-$R_2$-Imidazol-1-yl überführt, oder

c) in einer Verbindung der Formel

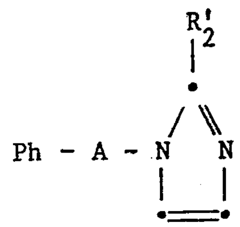

(IV),

worin A für eine in $-CH(R_1)-$ überführbare Gruppierung steht und der Rest $R_2'$ einen in $R_2$ überführbaren Rest oder $R_2$ bedeutet, oder worin A für die Gruppierung $-CH(R_1)-$ steht und $R_2'$ einen in $R_2$ überführbaren Rest bedeutet, A in $-CH(R_1)-$ und/oder $R_2'$ in $R_2$ überführt, oder

d) eine Verbindung der Formel

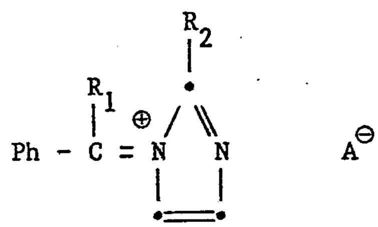

(V),

worin $A^{\ominus}$ ein Anion einer Protonensäure bedeutet, zu der entsprechenden Verbindung der Formel I reduziert, oder

e) eine Verbindung der Formel

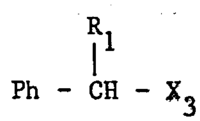

(VI),

worin $X_3$ für eine durch Cyclisierung in 2-$R_2$-Imidazol-1-yl überführbare Gruppe steht, cyclisiert, oder

f) eine Verbindung der Formel

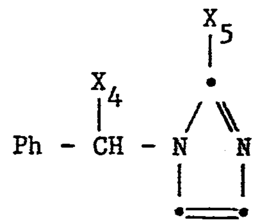

(VIIa)

mit einer Verbindung der Formel $R^o$-$X_6$ (VIIb) umsetzt, worin einer der Reste $X_4$ und $X_6$ reaktionsfähiges verestertes Hydroxy bedeutet und der andere für einen metallischen Rest steht, $X_5$ den Rest $R_2$ bedeutet und $R^o$ den Rest $R_1$ bedeutet, oder worin einer der Reste $X_5$ und $X_6$ reaktionsfähiges verestertes Hydroxy bedeutet und der andere für einen metallischen Rest steht, $X_4$ den Rest $R_1$ bedeutet und $R^o$ den Rest $R_2$ bedeutet, oder

g) zur Herstellung von Verbindungen der Formel I oder deren Salzen, worin Ph durch Niederalkoxy substituiertes Phenyl bedeutet, in einer Verbindung der Formel

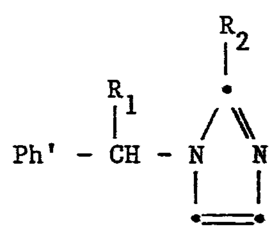

(VIII)

worin Ph' einen in Ph überführbaren Rest bedeutet, Ph' in Ph überführt,

wobei die in den Varianten a) bis g) aufgeführten Ausgangsverbindungen gegebenenfalls auch in Salzform vorliegen können, und, wenn erwünscht, eine erfindungsgemäss erhältliche Verbindung der Formel I in eine andere Verbindung der Formel I überführt und/oder gewünschtenfalls ein erhältliches Salz in die freie Verbindung der Formel I

oder in ein anderes Salz überführt und/oder gewünschtenfalls eine erfindungsgemäss erhältliche freie Verbindung der Formel I in ein Salz überführt und/oder gewünschtenfalls ein erhältliches Isomerengemisch in die einzelnen Komponenten auftrennt.

Die vor- und nachstehend in den Varianten a) bis g) beschriebenen Umsetzungen werden in an sich bekannter Weise durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -10° bis etwa +250°C, vorzugsweise von etwa 20° bis etwa 150°C, und, falls erforderlich, in einem geschlossenen Gefäss, unter Druck, in einer Inertgasatmosphäre und/oder unter wasserfreien Bedingungen arbeitet.

Das vor- und nachstehend aufgeführte Ausgangsmaterial der Formeln IIa und IIb, III, IV, V, VI, VIIa und VIIb sowie VIII, das für die Herstellung der Verbindungen der Formel I entwickelt wurde, ist zum Teil bekannt oder kann ebenfalls nach an sich bekannten Methoden, z.B. analog den vor- und nachstehend beschriebenen Verfahrensvarianten, hergestellt werden.

Variante a):
Reaktionsfähiges verestertes Hydroxy bedeutet insbesondere mit einer starken anorganischen Säure oder organischen Sulfonsäure verestertes Hydroxy, beispielsweise Halogen, wie Chlor, Brom oder Jod, Sulfonyloxy, wie Hydroxysulfonyloxy, Halogensulfonyloxy, z.B. Fluorsulfonyloxy, gegebenenfalls, z.B. durch Halogen, substituiertes Niederalkansulfonyloxy, z.B. Methan-oder Trifluormethan-sulfonyloxy, Cycloakansulfonyloxy, z.B. Cyclohexansulfonyloxy, oder gegebenenfalls, z.B. durch Niederalkyl oder Halogen, substituiertes Benzolsulfonyloxy, z.B. p-Bromphenyl- oder p-Toluolsulfonyloxy.

Ein metallischer Rest ist beispielsweise ein Alkalimetall-, insbesondere Lithiumrest, ferner ein Kupfer I- oder sich von Lithiumcupraten ableitender Lithium-Kupfer (I)-Rest.

Die Umsetzung wird insbesondere in Gegenwart eines Kondensationsmittels, wie einer geeigneten Base, durchgeführt.

Als Basen kommen beispielsweise Alkalimetall-hydroxide, -hydride, -amide, -alkanolate, -carbonate, -triphenylmethylide, -diniederalkylamide, -aminoalkylamide oder -niederalkylsilylamide, Naphthalinamine, Niederalkylamine, basische Heterocyclen, Ammoniumhydroxid sowie carbocyclische Amine in Frage. Beispielhaft seien Natrium-hydroxid, -hydrid, -amid, Kalium-tert-butylat, -carbonat, Lithium-triphenylmethylid, -diisopropylamid, Kalium-3-(aminopropyl)-amid, -bis-(trimethylsilyl)-amid, Dimethylaminonaphthalin, Di- oder Triethylamin, Pyridin, Benzyl-trimethyl-ammoniumhydroxid, 1,5-Diaza-bicyclo[4.3.0]non-5-en (DBN) sowie 1,8-Diaza-bicyclo[5.4.0]undec-7-en (DBU) genannt.

Bedeutet $X_2$ Wasserstoff, ist der Zusatz von zusätzlicher Base nicht erforderlich, wenn die als Base wirkende Verbindung der Formel IIb im Ueberschuss eingesetzt wird. Die Umsetzung mit Verbindungen der Formel IIb, wenn $X_2$ für einen metallischen Rest steht, erfolgt vorteilhaft ohne Basenzusatz.

Bevorzugt wird die Umsetzung mit solchen Verbindungen der Formel IIb durchgeführt, worin $X_2$ für Wasserstoff steht.

<u>Variante b):</u>
Ein in 2-$R_2$-Imidazol-1-yl überführbarer Rest Het kann z.B. 3-$R_2$-Pyrazol-1-yl sein.

Dessen Ueberführung in 2-$R_2$-Imidazol-1-yl kann beispielsweise durch Photoisomerisierung, z.B. durch Bestrahlen, wie mit einer Hochdruck-Quecksilberlampe, erfolgen.

Ein in 2-$R_2$-Imidazol-1-yl überführbarer Rest Het kann ebenso 2-$R_2$-Imidazolin-1-yl, insbesondere 2-$R_2$-imidazol-2-in-1-yl, bedeuten, welcher z.B. durch Dehydrierung in 2-$R_2$-Imidazol-1-yl übergeführt werden kann.

Für die Dehydrierung verwendet man geeignete Dehydrierungsmittel, beispielsweise Nebengruppenelemente, vorzugsweise solche der VIII. Nebengruppe des Periodensystems, z.B. Palladium, Raney-Nickel oder Platin, oder entsprechende Edelmetallderivate, z.B. Platinoxid oder Ruthenium-triphenylphosphid-chlorid, wobei die Mittel auf geeigneten Trägermaterialien, wie Kohle, aufgezogen sein können. Weitere bevorzugte Dehydrierungsmittel sind beispielsweise Chinone, wie p-Benzochinone, z.B. Tetrachlor-p-benzochinon oder 2,3-Dichlor-5,6-dicyano-p-benzochinon, sowie Phenanthren-9,10-chinon. Ferner können N-Halogensuccinimide, wie N-Chlorsuccinimid, Manganverbindungen, wie Bariummanganat oder Mangandioxid, und Schwefel- sowie Selenderivate, wie Schwefel, Selen, Selendioxid oder Diphenylselenium-bis-trifluoracetat, verwendet werden.

Variante c):

Ein in die Gruppierung $-CH(R_1)-$ überführbares Strukturelement A steht beispielsweise für die Gruppierung $-C(R_1')(Z_o)-$, wobei $R_1'$ entweder den Rest $R_1$ bedeutet, der einen in Wasserstoff überführbaren Rest $Z_1$ aufweist, und $Z_o$ Wasserstoff ist, oder $R_1'$ für $R_1$ steht und $Z_o$ ein in Wasserstoff überführbarer Rest $Z_1$ ist, oder wobei $R_1'$ und $Z_o$ gemeinsam Niederalkenyliden oder eine tautomere Form davon bilden. Ebenso sind in $R_2$ überführbare Reste $R_2'$ durch einen in Wasserstoff überführbaren Rest $Z_1$ substituierte Niederalkylreste.

$Z_1$ kann z.B. durch Reduktion in Wasserstoff überführt werden und bedeutet dementsprechend beispielsweise Hydroxy, verethertes Hydroxy, wie Niederalkoxy, Halogen, Sulfonyloxy, wie gegebenenfalls,

z.B. durch Halogen, substituiertes Niederalkansulfonyloxy, Cycloniederalkansulfonyloxy oder gegebenenfalls, z.B. durch Niederalkyl oder Halogen, substituiertes Benzolsulfonyloxy, Mercapto oder verethertes Mercapto, wie Niederalkylthio.

Die reduktive Ueberführung von A in die Gruppierung $-CH(R_1)-$ bzw. von $R_2'$ in $R_2$ erfolgt in an sich bekannter Weise, z.B. mit Hilfe eines Reduktionsmittels, beispielsweise durch Hydrierung in Gegenwart eines Hydrierungskatalysators, durch Reduktion mit einem Hydrid übertragenden Reagenz oder durch Reduktion mit einem metallischen Reduktionssystem aus Metall und protonenabspaltendem Mittel.

Als Hydrierungskatalysatoren kommen z.B. Elemente der VIII. Nebengruppe des Periodensystems der Elemente oder deren Derivate, wie Palladium, Platin, Platinoxid, Ruthenium, Rhodium, Tris-(triphenylphosphin)-rhodium(I)halogenid, z.B. -chlorid, oder Raney-Nickel, die gegebenenfalls auf einem Trägermaterial, wie Aktivkohle, Alkalimetallcarbonat bzw. -sulfat oder einem Kieselgel, aufgezogen sind, in Frage. Als Hydrid-übertragende Reagenzien kommen beispielsweise geeignete Leichtmetallhydride, insbesondere Alkalimetallaluminiumhydride bzw. -borhydride, wie Lithiumaluminiumhydrid, Lithiumtriethylborhydrid, Natriumborhydrid, Natriumcyanoborhydrid, oder Zinnhydride, wie Triethyl- oder Tributylzinnhydrid, oder Diboran in Frage. Der Metallbestandteil des metallischen Reduktionssystems ist beispielsweise ein unedles Metall, wie Alkali-oder Erdalkalimetall, z.B. Lithium, Natrium, Kalium, Magnesium oder Calcium, oder Uebergangsmetall, z.B. Zink, Zinn, Eisen oder Titan, während als protonenabspaltendes Mittel z.B. Protonsäuren, wie Salz- oder Essigsäure, Niederalkanole, wie Ethanol, und/oder Amine bzw. Ammoniak, in Frage kommen. Solche Systeme sind beispielsweise Natrium/Ammoniak, Zink/Salz- oder Essigsäure oder Zink/Ethanol.

In bevorzugten Ausführungsformen dieses Verfahrens werden beispielsweise Hydroxy oder gegebenenfalls verethertes Mercapto durch

katalytische Hydrierung, z.B. in Gegenwart von Raney-Nickel, und Halogen oder Sulfonyloxy, z.B. durch Hydrid-übertragende Reagenzien, durch Tributylzinnhydrid, Lithiumaluminiumhydrid oder Natriumcyanoborhydrid oder Natrium/Ammoniak reduziert.

Hydroxy kann ferner durch Behandlung mit Phosphor (rot) und Iodwasserstoff bzw. Iod durch Wasserstoff ersetzt werden.

Bevorzugt werden in benzylischer Stellung befindliche durch Wasserstoff ersetzbare Reste $Z_1$ durch Wasserstoff ersetzt.

$Z_1$ kann ferner für Carboxy stehen. Die Decarboxylierung entsprechender Verbindungen der Formel IV kann üblicherweise bei erhöhten Temperaturen, beispielsweise ab einer Temperatur von etwa 50°C, insbesondere in einem Temperaturbereich von etwa 100° bis etwa 300°C, durchgeführt werden. Die Decarboxylierung kann beispielsweise durch die Gegenwart von Basen, wie hochsiedenden Stickstoffbasen, z.B. Collidin, und/oder in Gegenwart von Edelmetallen, wie Kupfer oder Kupferbronze, unterstützt werden.

In erster Linie geht man von solchen Verbindungen der Formel IV aus, die lediglich einen in Wasserstoff überführbaren Rest $Z_1$ aufweisen.

Variante d):
Die Reduktion der Iminiumsalze der Formel V zu den tertiären Aminen der Formel I erfolgt beispielsweise mit Hilfe eines Reduktionsmittels, z.B. eines solchen der in Variante c) aufgeführten Art. Beispielhaft für geeignete Möglichkeiten der Reduktion sei die katalytische Hydrierung, Behandlung mit einem Hydrid-übertragenden Reagenz, z.B. Natriumborhydrid, oder die Verwendung des metallischen Reduktionssystems Zink/Salzsäure genannt.

Als Reduktionsmittel kann ferner Ameisensäure verwendet werden.

Variante e):

Eine durch Cyclisierung in 2-$R_2$-Imidazol-1-yl überführbare Gruppierung $X_3$ steht beispielsweise für die Gruppe der Formel

$$- N = CH - CH_2 - NH - CO - R_2 \qquad (VIa).$$

Die Cyclisierung der Gruppe VIa erfolgt beispielsweise durch übliches Behandeln entsprechender Verbindungen der Formel VII mit einem sauren Mittel, insbesondere einem Mineralsäureanhydrid, wie einem Phosphoroxyhalogenid, z.B. -chlorid, oder Phosphorpentahalogenid, z.B. -chlorid. Besonders geeignet ist das System Triphenylphosphin/Hexachlorethan/Triethylamin. In erster Linie verfährt man analog der von W. Steglich et al., Liebigs Ann. Chem. 1978, 1916-1927 beschriebenen Weise.

$X_3$ kann weiterhin für eine Gruppe der Formel

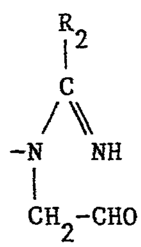 (VIb) bzw. 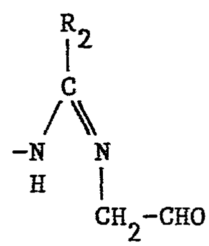 (VIb')

stehen, wobei die Formylgruppe auch in acetalisierter Form, beispielsweise als mit einem Alkohol, wie Niederalkanol bzw. Niederalkandiol, acetalisiertes Formyl, vorliegen kann.

Die Cyclisierung kann beispielsweise in Gegenwart eines sauren Mittels, beispielsweise einer Protonsäure, wie einer Mineralsäure, z.B. Halogenwasserstoff-, Schwefel- oder Polyphoshorsäure, einer Sulfonsäure, z.B. Trifluormethan- oder p-Toluolsulfonsäure, oder einer starken Carbonsäure, z.B. gegebenenfalls substituierten Niederalkancarbonsäure, z.B. Eisessig oder Trifluoressigsäure, erfolgen. Ferner eignen sich beispielsweise die vorstehend genannten Mineralsäureanhydride.

Variante f):
Reaktionsfähiges verestertes Hydroxy $X_4$ bzw. $X_5$ bzw. $X_6$ hat beispielsweise die in Variante a) für $X_1$ angegebene Bedeutung, während ein metallischer Rest $X_4$ bzw. $X_5$ bzw. $X_6$ z.B. einen Alkalimetall-, wie Lithium-, Natrium- oder Kaliumrest, einen Magnesiumhalogenid-, wie -bromidrest, einen Kupfer- oder sich von Lithiumcupraten ableitenden Lithium-Kupfer-Rest bedeutet.

In einer Abwandlung des Verfahrens kann man beispielsweise von einer Verbindung der Formel VIIa ausgehen, worin $X_4$ und $X_5$ jeweils Wasserstoff bedeuten, und diese mit einer starken Base, wie Butyllithium, behandeln und die so erhältliche Verbindung der Formel VIIa, worin $X_4$ und $X_5$ jeweils für einen metallischen Rest, wie Lithium, stehen, mit mindestens 2 Mol einer Verbindung der Formel VIIb umsetzen. Dabei kann man zunächst in situ eine Verbindung der Formel VIIa erhalten, worin einer der Reste $X_4$ und $X_5$ für einen metallischen Rest und der andere für $R_1$ bzw. $R_2$ steht, die unter den Reaktionsbedingungen direkt weiter zu der entsprechenden Verbindung der Formel I reagiert.

Vorteilhaft wird diese Umsetzung zunächst bei tiefen Temperaturen, z.B. in einem Temperaturbereich von -78°C bis Raumtemperatur, durchgeführt.

Variante g): Ph' bedeutet beispielsweise durch Hydroxy substituiertes Phenyl.
Derartige Verbindungen der Formel VIII können durch Veretherung mit einem Niederalkylierungsmittel in die Verbindungen der Formel I übergeführt werden. Zu den Niederalkylierungsmitteln zählen beispielsweise Niederalkanole oder reaktionsfähige Ester davon, wie entsprechende Halogen-, wie Chlor-, Brom- oder Jod-, Sulfonyloxy-, wie Hydroxysulfonyloxy-, Halogensulfonyloxy-, z.B. Fluorsulfonyloxy-, gegebenenfalls, z.B. durch Halogen, substituierte Niederalkansulfonyloxy-, z.B. Methan- oder Trifluormethansulfonyloxy-, Cycloalkansulfonyl-, z.B. Cyclohexansulfonyloxy-, oder gegebenenfalls, z.B. durch Niederalkyl oder Halogen, substituierte Benzolsulfonyloxyderivate, z.B. p-Bromphenyl-oder p-Toluolsulfonyloxy-Derivate. Ebenso kommen als Niederalkylierungsmittel z.B. Diniederalkylsulfat, Diazoniederalkan, Triniederalkylsulfonium-, Triniederalkylselenium-, Triniederalkyloxosulfonium- oder Triniederalkylanilinium-hydroxid, ferner Pentaniederalkoxyphosphin, in Betracht.

Bei der Verwendung von reaktionsfähigen Estern von Niederalkanolen oder Diniederalkylsulfaten als Niederalkylierungsmitteln erfolgt die Veretherung insbesondere in Gegenwart einer der vorstehend genannten Basen, während die Umsetzung mit einem Diazoniederalkan gegebenenfalls in Gegenwart einer Lewis-Säure durchgeführt wird. Lewis-Säuren sind beispielsweise Halogenide von Bor, Aluminium, Zinn(II), Antimon(III), Arsen(III), Silber(I), Zink(II) und Eisen(III).

Die Veretherung mit Hilfe eines Niederalkanols wird beispielsweise in Gegenwart einer starken Säure oder, unter wasserfreien Bedingungen, eines Dehydratisierungsmittels durchgeführt.

Als starke Säuren seien insbesondere starke Protonsäuren, beispielsweise Mineralsäuren, wie Halogenwasserstoffsäuren, Schwefel- oder eine Phosphorsäure, starke Carbonsäuren, wie eine gegebenenfalls, z.B. durch Halogen, substituierte Niederalkancarbonsäure bzw. Benzoesäure, z.B. Eisessig oder Trifluoressigsäure, oder Sulfonsäuren, wie gegebenenfalls, z.B. durch Halogen, substituierte Niederalkansulfonsäure oder gegebenenfalls, z.B. durch Halogen oder Niederalkyl, substituierte Benzolsulfonsäure, z.B. p-Toluolsulfonsäure, genannt.

Geeignete Dehydratisierungsmittel sind beispielsweise Carbodiimide, z.B. N,N'-Diniederalkyl- oder N,N'-Dicycloalkyl-carbodiimid, wie N,N'-Diethyl-, N,N'-Diisopropyl- oder N,N'-Dicyclohexyl-carbodiimide, vorteilhaft unter Zusatz von N-Hydroxysuccinimid oder gegebenenfalls, z.B. durch Halogen, Niederalkyl oder Niederalkoxy,

substituiertes 1-Hydroxy-benzotriazol oder N-Hydroxy-5-norbornen-2,3-dicarboxamid, N,N'-Diimidazolcarbonyl, eine geeignete Phosphoryl- bzw. Phosphinverbindung, z.B. Diethylphosphonylcyanid, Diphenylphosphonylazid oder Triphenylphosphin-disulfid, ein 1-Niederalkyl-2-halogen-pyridinium-halogenid, z.B. 1-Methyl-2-chlor-pyridinium-iodid, ein geeignetes 1,2-Dihydrochinolin, z.B. N-Ethoxycarbonyl-2-ethoxy-1,2-dihydrochinolin, oder 1,1'-(Carbonyldioxy)-dibenzotriazol.

Ph' bedeutet beispielsweise ebenso eine Diazoniumgruppierung $-N_2^{\oplus} A^{\ominus}$ aufweisendes Phenyl, wobei $A^{\ominus}$ ein Anion einer starken Protonsäure bedeutet.

Zur Ueberführung derartiger Verbindungen der Formel VIII in entsprechende Verbindungen der Formel I wird die Diazoniumgruppierung $-N_2^{\oplus} A^{\ominus}$ durch Niederalkoxy substituiert, insbesondere durch Behandeln mit einem Niederalkanol.

In einer vorteilhaften Modifikation dieser Verfahrensvariante können die Verbindungen der Formel VIII in situ gebildet werden und unter den Reaktionsbedingungen ohne Isolierung zu den Verbindungen der Formel I weiterreagieren. Dabei geht man zunächst von einer Verbindung der Formel VIII aus, worin Ph' eine Aminogruppe aufweisendes Phenyl bedeutet, diazotiert diese mit Nitriten, wie Alkalimetallnitriten, oder Nitroniederalkanen in Gegenwart von Protonensäuren, z.B. solchen der vorstehend genannten Art, und setzt die in situ gebildeten Verbindungen der Formel VIII, worin Ph' eine Diazoniumgruppierung $N_2^{\oplus} A^{\ominus}$ aufweisendes Phenyl bedeutet, ohne deren Isolierung mit einem Niederalkanol um. Vorteilhaft wird für diese Umsetzung eine Reaktionstemperatur von etwa -10° bis etwa +40°C gewählt.

Die Erfindung betrifft vor allem die in den Ausführungsbeispielen beschriebenen Herstellungsverfahren.

Eine erfindungsgemässe Verbindung kann nach an sich bekannten Methoden in eine andere erfindungsgemässe Verbindung übergeführt werden.

So kann beispielsweise in den Phenylring Ph ein Niederalkylrest eingeführt werden, indem beispielsweise mit einem reaktionsfähigen Ester eines Niederalkanols in Gegenwart einer Lewissäure alkyliert wird (Friedel-Crafts-Alkylierung).

Die Herstellung der in den Varianten a) bis g) beschriebenen Ausgangsmaterialien erfolgt unter Anwendung an sich bekannter Methoden.

So kann beispielsweise das Ausgangsmaterial der Formel III durch Umsetzung von Verbindungen der Formel IIa mit Verbindungen der Formel $X_2$-Het (IIIa) analog der in Variante a) beschriebenen Verfahrensweise hergestellt werden.

In analoger Weise kann man ebenso zu Ausgangsverbindungen der Formeln IV, VIIa bzw. VIII gelangen, z.B. durch Umsetzung von Verbindungen der Formeln Ph-A-$X_1$ (IVa) und (IIb) [führt zu Verbindungen der Formel IV] bzw. Ph-CH($X_4$)-$X_1$ (VIIc) und

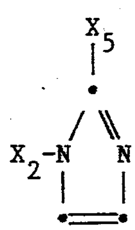

(VIId) [führt zu Verbindungen der Formel VIIa]

sowie Ph'-CH($R_1$)-$X_1$ (IIIa) und (IIb) [führt zu Verbindungen der Formel VIII].

Verbindungen der Formel VI, worin $X_3$ für die Gruppierung der Formel VIa steht, sind beispielsweise erhältlich durch säurekatalysierte Reaktion von Verbindungen der Formeln

$R_2 - CO - NH - CH_2 - CH(=O)$ (VIc)

mit Verbindungen der Formel

$H_2N - CH(R_1) - Ph$ (VId).

Verbindungen der Formel VI, worin $X_3$ für die Gruppierung der Formel VIb bzw. VIb' steht, sind beispielsweise durch Kondensation von Verbindungen der Formel

$R_2 - C(=NH) - X_8$ (VIe),

worin $X_8$ für eine Abgangsgruppe, wie Niederalkoxy oder Amino, steht, und Verbindungen der Formel

$Ph - CH(R_1) - NH - CH_2 - CH(=O)$ (VIf)

zugänglich, wobei die sekundäre Aminogruppe auch zusätzlich einen Acylrest, wie Niederalkanoyl, z.B. Acetyl, aufweisen kann.

Eine weitere Variante zur Herstellung einer Verbindung der Formel VI, worin $X_3$ die Gruppierung der Formel VIb bzw. VIb' bedeutet, besteht z.B. in der Umsetzung einer Verbindung der Formel VId mit einer Verbindung der Formerl VIe und Kondensation der so erhältlichen Verbindung der Formel $Ph-CH(R_1)-NH-C(R_2)=NH$ (VIg) mit einem Monohalogenacetaldehyd oder insbesondere einem Acetal davon. Ebenso kann man eine Verbindung der Formel $Ph-CH(R_1)-N=(R_2)-X_8$ (VIh) mit einem Acetal des Aminoacetaldehyds umsetzen.

Eine Verbindung der Formel VI, worin $X_3$ für die Gruppierung VIb bzw. VIb' steht, kann insbesondere in situ gebildet werden und unter den Reaktionsbedingungen direkt zu der entsprechenden Verbindung der Formel I weiterreagieren.

Salze von Verbindungen der Formel (I) können in an sich bekannter Weise hergestellt werden. So erhält man beispielsweise Säureadditionssalze von Verbindungen der Formel (I) durch Behandeln mit einer Säure oder einem geeigneten Ionenaustauscherreagenz. Salze können in üblicher Weise in die freien Verbindungen überführt werden, Säureadditionssalze z.B. durch Behandeln mit einem geeigneten basischen Mittel.

Je nach Verfahrensweise bzw. Reaktionsbedingungen können die erfindungsgemässen Verbindungen mit salzbildenden, insbesondere basischen Eigenschaften, in freier Form oder in Form von Salzen erhalten werden.

Infolge der engen Beziehung zwischen der neuen Verbindung in freier Form und in Form ihrer Salze sind im vorausgegangenen und nachfolgend unter der freien Verbindung oder ihren Salzen sinn- und zweckgemäss gegebenenfalls auch die entsprechenden Salze bzw. die freie Verbindung zu verstehen.

Die neuen Verbindungen einschliesslich ihrer Salze von salzbildenden Verbindungen können auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate getrennt aufgetrennt werden, beispielsweise durch fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, Chromatographie an chiralen Adsorbentien mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein- oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes, und/oder seiner Racemate bzw. Antipoden, verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, die speziell für die Herstellung der erfindungsgemässen Verbindungen entwickelt wurden, ihre Verwendung und Verfahren zu ihrer Herstellung bilden ebenfalls einen Gegenstand der Erfindung, wobei die Variablen $R_1$, $R_2$ und Ph die für die jeweils bevorzugten Verbindungsgruppen der Formel I angegebenen Bedeutungen haben.

Die Erfindung betrifft ebenfalls die Verwendung der Verbindungen der Formel (I) oder von pharmazeutisch verwendbaren Salzen von solchen Verbindungen mit salzbildenden Eigenschaften, insbesondere als pharmakologische, in erster Linie antidepressiv wirksame, Wirksubstanzen. Dabei kann man sie, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Antidepressiva zur Behandlung von Depressionen, verwenden.

Die Erfindung betrifft gleichfalls pharmazeutische Präparate, die die erfindungsgemässen Verbindungen oder pharmazeutisch verwendbare Salze derselben als Wirkstoffe enthalten, sowie Verfahren zu ihrer Herstellung.

Bei den erfindungsgemässen pharmazeutischen Präparaten, welche die erfindungsgemässe Verbindung oder pharmazeutisch verwendbare Salze davon enthalten, handelt es sich um solche zur enteralen, wie oralen ferner rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffs hängt von dem Alter und dem individuellen Zustand, sowie von der Applikationsweise ab.

Die Wirksubstanzen können ferner mit Hilfe von transdermalen therapeutischen Systemen (TTS), die der kontrollierten perkutanen Wirkstoffzufuhr für eine systemische Behandlung dienen, beispielsweise in Form eines Heftpflasters von abgerundeter Form mit einer Grösse von etwa 2 bis 50 $cm^2$ auf die Haut gebracht werden. Die Wirkstoffabgabe kann beispielsweise über einen Zeitraum von etwa 24 Stunden bis zu einer Woche erfolgen.

Derartige Transdermalsysteme sind mehrschichtig aufgebaut und bestehen z.B. von aussen nach innen aus einer undurchlässigen Abdeckfolie, einem Wirkstoffreservoir, einer Adhäsiv- oder Kleberschicht sowie einer vor der Applikation zu entfernenden Abziehfolie. Bei den entsprechenden Matrix- oder Monolithsystemen ist der Wirkstoff in einer Polymerschicht verteilt, aus der er durch Diffusion freigesetzt wird. Ferner können auch membrankontrollierte Systeme eingesetzt werden, bei denen sich zwischen dem Wirkstoffreservoir und der Haut eine die Diffusionsgeschwindigkeit bestimmende semipermeable oder mikroporöse Kontrollmembran befindet. Für die Dosierung ist die Menge des pro Zeiteinheit aufgenommenen Wirkstoffs z.B. von der Grösse der Kontaktfläche zwischen Wirkstoffreservoir und Haut abhängig.

Die neuen pharmazeutischen Präparate enthalten z.B. von etwa 10% bis etwa 80%, vorzugsweise von eta 20% bis etwa 60%, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphat, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragaganth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliess-, Regulier-und Schmiermitel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet oder wässerige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Dosierung des Wirkstoffs hängt von der Warmblüter-Spezies, dem Alter und dem individuellen Zustand sowie der Applikationsweise ab. Im Normalfall ist für einen etwa 75 kg schweren Warmblüter bei oraler Applikation eine ungefähre Tagesdosis von etwa 100 bis etwa 500 mg, insbesondere 200 bis etwa 300 mg, vorteilhaft im mehreren gleichen Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1: 24,4 g (0,2 Mol) 1-Phenyl-ethanol und 27,6 g (0,24 Mol) Methansulfochlorid werden in 250 ml Toluol gelöst und bei 10° im Verlaufe von 30 Minuten wird eine Lösung von 28,2 g (0,28 Mol) Triethylamin in 50 ml Toluol unter Rühren eingetropft. Das Reaktionsgemisch wird 12 Stunden bei Raumtemperatur gerührt und anschliessend das ausgefallene Triethylamin-hydrochlorid abfiltriert. Der Rückstand wird mit Toluol ausgewaschen und die vereinigten Filtrate im Wasserstrahlvakuum zur Trockene eingedampft. Das erhaltene Oel wird unter Rühren bei 95° im Verlaufe von 15 Minuten zu einer Lösung von 50 g (0,6 Mol) 2-Methylimidazol zugetropft und 6 Stunden bei 100° gehalten. Das Reaktionsgemisch wird mit 200 ml Ether und 200 ml Wasser versetzt, die organische Phase abgetrennt und 3 mal mit Wasser ausgewaschen. Die organischen Phasen werden 3 mal mit je 100 ml Salzsäure 2n ausgeschüttelt, die wässrigen, sauren Phasen mit konz. Natronlauge alkalisch gestellt und wiederum mit Ether ausgeschüttelt. Die organischen Phasen werden über Magnesiumsulfat getrocknet und das Lösungsmittel am Wasserstrahlvakuum entfernt. Das als Rohprodukt erhaltene Oel wird am Hochvakuum bei 120° (13,3 Pa) destilliert und ergibt nach Umkristallisation aus Cyclohexan reines 1-(1-Phenyl-ethyl)-2-methyl-imidazol, welches bei 84-85° schmilzt. 22,8 g (0,12 Mol) der erhaltenen Base werden in 100 ml Essigsäureethylester gelöst und mit alkoholischer Salzsäure

sauer gestellt (pH ~ 1). Nach Zugabe von 25 ml Ether wird der ausgefallene Niederschlag abfiltriert, mit Ether gewaschen und am Vakuum bei 60° getrocknet. Das erhaltene reine 1-(1-Phenylethyl)-2-methyl-imidazol-hydrochlorid schmilzt bei 229-229,5°.

Beispiel 2: In analoger Weise wie in Beispiel 1 beschrieben wird aus 40,86 g (0,3 Mol) 1-(p-Methyl-phenyl)-ethanol und 73,9 g (0,9 Mol) 2-Methylimidazol öliges 1-[1-(p-Methyl-phenyl)-ethyl]-2-methyl-imidazol erhalten. Das hieraus hergestellte Hydrochlorid schmilzt bei 237-238°.

Beispiel 3: In analoger Weise wie in Beispiel 1 beschrieben werden aus 30,1 g (0,2 Mol) 1-(2,4-Dimethyl-phenyl)-ethanol und 49,3 g (0,6 Mol) 2-Methylimidazol 21,3 g öliges 1-[1-(2,4-Dimethyl-phenyl)-ethyl]-2-methyl-imidazol als Rohprodukt erhalten. Das analog hergestellte Hydrochlorid schmilzt bei 238-239°.

Das als Ausgangsmaterial benötigte 1-(2,4-Dimethyl-phenyl)-ethanol wird wie folgt hergestellt:

44,5 g (0,3 Mol) 2,4-Dimethylacetophenon werden in 200 ml Isopropanol gelöst und bei 60° unter Rühren eine eiskalte Lösung von 5,7 g (0,15 Mol) Natriumborhydrid in 50 ml Wasser langsam eingetropft. Das Reaktionsgemisch wird 4 Stunden auf Rückflusstemperatur erhitzt, abgekühlt und mit 200 ml 1-molarer Natriumdihydrogenphosphatlösung versetzt. Das Isopropanol wird am Wasserstrahlvakuum weitgehend entfernt und der Rückstand mit Ether ausgeschüttelt. Die organischen Phasen werden mit Sole ausgewaschen, über Magnesiumsulfat getrocknet und das Lösungsmittel am Wasserstrahlvakuum entfernt. Der erhaltene Rückstand wird im Wasserstrahlvakuum destilliert und ergibt 1-(2,4-Dimethyl-phenyl)-ethanol vom Kp.(1866,5 Pa) 120° [Ishizaka, Chem. Ber. 47, 2461, $Kp_{14}$ = 124-125°].

Beispiel 4: In analoger Weise wie in Beispiel 1 beschrieben wird aus 27,3 g (0,2 Mol) 1-[(2-Methyl-phenyl)-ethyl]-ethanol und 49,3 g (0,6 Mol) 2-Methylimidazol öliges 1-[1-(2-Methyl-phenyl)-ethyl]-2-methyl-imidazol als Rohprodukt erhalten. Das Hydrochlorid schmilzt bei 237-238°.

Das als Ausgangsmaterial benötigte 1-(2-Methyl-phenyl)-ethanol wird analog Beispiel 3 hergestellt (V. Auwers et al., Chem. Ber. 58, 46, $Kp_{20}$ = 107-108°).

Beispiel 5: 25 g (0,113 Mol) 1-(1-Phenyl-ethenyliden-1-yl)-2-methyl-imidazolhydrochlorid werden in 200 ml gesättigte Pottasche-Lösung gegeben und mit je 100 ml Ether 3 mal ausgeschüttelt. Die organischen Extrakte werden über Magnesiumsulfat getrocknet und am Vakuum zur Trockne eingedampft. Der erhaltene Rückstand wird in 500 ml Methanol gelöst und unter Zusatz von 1 g 5 % Palladiumkohle bei Raumtemperatur und Normaldruck hydriert. Der Katalysator wird abfiltriert, mit Methanol ausgewaschen und die vereinigten methanolischen Lösungen am Vakuum zur Trockne eingedampft. Der erhaltene ölige Rückstand wird aus Cyclohexan kristallisiert und ergibt reines 1-(1-Phenyl-ethyl)-2-methyl-imidazol vom Smp. 84-85°.

Das Ausgangsmaterial wird z.B. wie folgt erhalten:

a) 130 g (0,68 Mol) α-Chlor-phenylacetylchlorid werden bei 20-30° zu 500 ml Ethanol getropft und 3 Stunden bei 30-40° gerührt. Das Lösungsmittel wird am Vakuum entfernt und ergibt rohen 2-Chlor-2-phenyl-essigsäureethylester. Das erhaltene Produkt (137 g) wird in 2 l Toluol gelöst, und die Lösung wird mit 170 g (2,07 Mol) 2-Methylimidazol versetzt. Das Reaktionsgemisch wird unter Rühren während 12 Stunden bei 100° gehalten. Das erhaltene Reaktionsgemisch wird 4 mal mit 500 ml Wasser ausgeschüttelt, die wässerigen Phasen mit wenig Toluol nachgewaschen und die vereinigten organischen

Phasen über Magnesiumsulfat getrocknet. Nach Entfernen des Lösungsmittels am Vakuum wird roher öliger 2-Phenyl-2-(2-methyl-imidazol-1-yl)-essigsäureethylester erhalten. Die Verbindung destilliert bei 145° (26,66 Pa). Das reine Hydrochlorid schmilzt bei 157-159°.

b) 142,9 g (0,585 Mol) 2-Phenyl-2-(2-methyl-imidazol-1-yl)-essigsäureethylester werden in 1,5 l Isopropanol gelöst und unter Rühren bei 5° eine Lösung von 23 g (0,6 Mol) Natriumborhydrid in 150 ml Wasser im Verlaufe von 30 Minuten zugetropft. Das Gemisch wird ohne Kühlung 6 Stunden gerührt, wobei die Temperatur des Gemisches 45-50° erreicht. Das Gemisch wird kalt mit einem Gemisch aus konz. Salzsäure und Wasser (1:1) sauer gestellt (pH = 1-2) und das Lösungsmittel am Vakuum weitgehend entfernt. Der Rückstand wird in der Kälte mit gesättigter Pottaschelösung bis zur alkalischen Reaktion versetzt und mit je 300 ml Ether 3 mal ausgeschüttelt. Die vereinigten organischen Phasen werden über Magnesiumsulfat getrocknet und das Lösungsmittel am Vakuum entfernt. Der erhaltene ölige Rückstand wird aus Ether/Cyclohexan kristallisiert und ergibt reines 2-Phenyl-2-(2-methyl-imidazol-1-yl)-ethanol-(1) vom Smp. 114-116°.

105,3 g der erhaltenen Base werden in 300 ml Ethanol gelöst und mit alkoholischer Salzsäure bis zur sauren Reaktion versetzt. Nach Zugabe von 200 ml Essigester und ca. 200 ml Ether wird das ausgefallene Produkt abfiltriert, mit Essigester gewaschen und im Vakuum bei 60° getrocknet. Das reine 2-Phenyl-2-(2-methyl-imidazol-1-yl)-ethanol-hydrochlorid schmilzt bei 144-146°.

115 g (0,481 Mol) 2-Phenyl-2-(2-methyl-imidazol-1-yl)-ethanol-hydrochlorid werden in 1 l Chloroform gelöst und mit 120 g (1 Mol) Thionylchlorid 10 Stunden am Rückfluss gekocht. Das Reaktionsgemisch wird am Vakuum zur Trockne eingedampft und das Rohprodukt direkt weiterverwendet. 120,4 g des rohen 1-[2-(1-Chlor-2-phenyl-ethyl)]-2-methyl-imidazol-hydrohlorids werden in 1 l Chloroform gelöst und mit 152 g (1,0 Mol) 1,8-Diaza-bicyclo[5,4,0]-undec-7-en 12 Stunden bei Raumtemperatur gerührt.

Das Reaktionsgemisch wird am Vakuum vom Lösungsmittel befreit und mit je 500 ml Cyclohexan und Wasser versetzt. Die wässerige Phase wird abgetrennt und die organische Phase 3 mal mit je 100 ml Wasser gewaschen. Die organische Phase wird über Magnesiumsulfat getrocknet und das Lösungsmittel am Vakuum entfernt. Das rohe 1-(1-Phenyl-ethenyliden-1-yl)-2-methyl-imidazol wird als Oel erhalten. Die Base hat einen Siedepunkt vom Kp. 130° (6,66 Pa). 82 g der rohen Base werden in 200 ml Essigester gelöst und mit etwa 7n alkoholischer Salzsäure bis zur sauren Reaktion versetzt. Nach Zugabe von 50 ml Ether wird das ausgefallene Produkt abfiltriert, mit Ether gewaschen und im Vakuum bei 60° getrocknet. Das reine 1-(1-Phenyl-ethenyliden-1-yl)-2-methyl-imidazol-hydrochlorid schmilzt bei 258-260°.

Beispiel 6: 8,6 g (0,05 Mol) 1-(1-Phenyl-1-ethyl)-imidazol werden in 80 ml abs. Tetrahydrofuran gelöst und bei -70° 30 ml (2 Mol) n-Butyl-lithiumlösung in Hexan zugetropft. Das Gemisch wird 30 Minuten bei -70° gerührt und anschliessend 7,1 g (0,05 Mol) Methyljodid in 20 ml Tetrahydrofuran zugetropft. Die Kühlung wird entfernt, das Gemisch langsam auf Raumtemperatur gebracht und 2 Stunden bei dieser Temperatur belassen. Zur Aufarbeitung werden 25 ml 2n Natronlauge zugegeben und das Tetrahydrofuran am Vakuum weitgehend entfernt. Der Rückstand wird mit Ether ausgeschüttelt, die organischen Phasen über Magnesiumsulfat getrocknet und das Lösungsmittel am Vakuum entfernt. Der erhaltene ölige Rückstand wird in Essigester gelöst, mit etherischer Salzsäure bis zur sauren Reaktion versetzt und das ausgefallene Produkt abfiltriert. Nach Umkristallisation aus Isopropanol/Ether schmilzt das reine 1-(1-Phenyl-ethyl)-2-methyl-imidazol-hydrochlorid bei 228-229°.

Beispiel 7: In analoger Weise wie in Beispiel 1 wird aus 13,6 g (0,1 Mol) 1-[1-(3-Methyl-phenyl)-ethyl]-ethanol und 24,6 g (0,3 Mol) 2-Methylimidazol öliges 1-[1-(3-Methyl-phenyl)-ethyl]-2-methylimidazol als Rohprodukt erhalten. Das Hydrochlorid schmilzt bei 228-230°.

Beispiel 8: In analoger Weise wie in Beispiel 1 beschrieben, wird aus 13,6 g (0,1 Mol) 1-Phenyl-propanol-(1) und 25 g (0,3 Mol) 2-Methylimidazol rohes 1-[1-(Phenyl)-propyl]-2-methyl-imidazol als Oel erhalten. Das Hydrochlorid schmilzt bei 177-179°.

Beispiel 9: In analoger Weise wie in Beispiel 1 beschrieben, werden aus 15,0 g (0,1 Mol) 1-(2-Ethyl-phenyl)-ethanol und 25 g (0,3 Mol) 2-Methylimidazol rohes, öliges 1-[1-(2-Ethyl-phenyl)-ethyl]-2-methyl-imidazol erhalten. Das Hydrochlorid schmilzt bei 211-213°.

Beispiel 10: In analoger Weise wie in Beispiel 1 beschrieben, werden aus 15,0 g (0,1 Mol) 1-(2,6-Dimethyl-phenyl)-ethanol und 25 g (0,3 Mol) 2-Methylimidazol kristallisiertes 1-[1-(2,6-Dimethyl-phenyl)-ethyl]-2-methyl-imidazol vom Smp. 100-103° erhalten. Das Hydrochlorid schmilzt bei 265-267°.

Beispiel 11: 18,8 g (0,1 Mol) 1-[1-(Phenyl)-ethyl]-2-methyl-4,5-dihydroimidazol werden in 4000 ml Methylenchlorid gelöst und unter Rühren und Feuchtigkeitsausschluss 282 g (1,1 Mol) Bariummanganat beigefügt. Das Gemisch wird 24 Stunden am Rückfluss gerührt. Anschliessend werden 20 g Magnesiumsulfat zugegeben, von den festen Anteilen abfiltriert und der Rückstand mit Methylenchlorid ausgewaschen. Die organischen Filtrate werden am Vakuum zur Trockene eingedampft und das erhaltene Oel in Essigester gelöst. Nach Zugabe von etherischer Salzsäure bis zur sauren Reaktion wird das ausgefallene Hydrochlorid abfiltriert und aus Isopropanol-Ether umkristallisiert. Das reine 1-[1-(Phenyl)-ethyl]-2-methyl-imidazol-hydrochlorid schmilzt bei 228-229.

Das Ausgangsprodukt wird wie folgt erhalten:

a) N-[1-(Phenyl)-ethyl]-aminoethyl-amin

30 g (0,25 Mol) Acetophenon werden in 300 ml Methanol gelöst und nach Zugabe von 30 g (0,5 Mol) Ethylendiamin und 1,0 g Platin-Kohle mit Wasserstoff bei 20° und Normaldruck im Verlaufe von 22 Stunden

hydriert. Die erhaltene Lösung wird vom Katalysator abfiltriert, das Lösungsmittel am Vakuum entfernt und der Rückstand am Wasserstrahlvakuum destilliert. Das als Oel erhaltene N-[1-(Phenyl)-ethyl]-aminoethylamin siedet bei 115-118° (1466,5 Pa).

b) 16,4 g (0,1 Mol) N-[1-(Phenyl)-ethyl]-aminoethylamin werden in 100 ml Ethanol gelöst und bei 0° 16,0 g (0,1 Mol) Acetamidsäureethylester-hydrochlorid eingetragen. Das Gemisch wird 3 Stunden bei 0° gerührt und das Lösungsmittel am Vakuum entfernt. Das erhaltene Oel wird mit Wasser und Ether ausgeschüttelt, der etherische Extrakt wird über Magnesiumsulfat getrocknet und das Lösungsmittel am Vakuum entfernt. Der Rückstand wird in Essigester gelöst und mit Oxalsäure bis zur sauren Reaktion versetzt. Nach Zugabe von etwas Ether wird das ausgefallene Produkt abfiltriert und aus Isopropanol-Ether umkristallisiert. Das reine 1-[1-(Phenyl)-ethyl]-2-methyl-4,5-dihydroimidazol-oxalat schmilzt bei 80-84°.

Zur weiteren Umsetzung werden 29 g des Oxalates mit 1n Kalilauge und Ether in die freie Base überführt. Es wird reine Base erhalten, welche bei 130° (1,33 Pa) siedet.

<u>Beispiel 12</u>: 5,0 g (0,0409 Mol) R-(+)-1-Phenylethanol ($[\alpha]_D^{22}$ = +39,5°) und 5,2 g (0,055 Mol) Methansulfochlorid werden in 100 ml Ether gelöst, auf 0° gekühlt und bei dieser Temperatur eine Lösung von 5,1 g (0,060 Mol) Triethylamin in 50 ml Ether eingetropft. Das Gemisch wird 1 Stunde bei 0° gerührt und vom ausgefallenen Niederschlag abfiltriert. Das Filtrat wird am Wasserstrahlvakuum möglichst schonend eingedampft, der Rückstand in 20 ml Toluol gelöst und bei 90° zu einer Lösung von 8,2 g (0,1 Mol) 2-Methylimidazol getropft. Das Gemisch wird 1 Stunde auf 90° erwärmt.
Zur Aufarbeitung wird das Gemisch mit Ether verdünnt und mit Wasser und anschliessend mit 3 mal 50 ml 2n Salzsäure ausgeschüttelt. Die salzsauren Extrakte werden mit konzentrierter Natronlauge alkalisch gestellt und mit Ether ausgezogen. Die erhaltenen etherischen Lösungen werden über Magnesiumsulfat getrocknet und zur Trockene

eingeengt. Der verbleibende Rückstand wird 2 mal aus Cyclohexan kristallisiert und ergibt reines linksdrehendes 1-(1-Phenyl-ethyl)-2-methyl-imidazol vom Smp. 113-115° $[\alpha]_D^{20}$ = -15° ± 1°. (Enantiomerenreinheit etwa 95 % gemäss NMR). Das Hydrochlorid schmilzt bei 243-244°

Beispiel 13: In gleicher Weise wie in Beispiel 11 gezeigt wurde, werden aus 5,0 g S(-)-1-Phenylethanol ($[\alpha]_D^{20}$ = -41,3°) rechtsdrehendes 1-[1-(Phenyl)-ethyl]-2-methyl-imidazol vom Smp. 113-115 $[\alpha]_D^{20}$ = +15° (± 1°) erhalten. Das Hydrochlorid schmilzt bei 243-244°.

Beispiel 14: In analoger Weise wie in Beispiel 1 beschrieben, werden aus 20,2 g (0,1 Mol) 1-(3-Methoxyphenyl)-ethanol und 25 g (0,3 Mol) 2-Methylimidazol 1-[1-(3-Methoxy-phenyl)-ethyl]-2-methylimidazol als Oel erhalten. Das Hydrochlorid schmilzt bei 197-199°.

Beispiel 15: 6,0 g (0,0297 Mol) 1-[1-(2-Hydroxy-phenyl)-ethyl]-2-methyl-imidazol und 25 ml Hexametapol werden in 25 ml Tetrahydrofuran suspendiert und bei 20° unter Rühren portionenweise 1,3 g (0,030 Mol) Natriumhydridsuspension (55 %) zugegeben. Das Gemisch wird 2 Stunden bei 20° gerührt. Anschliessend wird eine Lösung von 4,25 g (0,030 Mol) Methyljodid in 25 ml Hexametapol eingetropft und das Gemisch 1 Stunde bei 45° gehalten.

Das Reaktionsgemisch wird auf 200 ml Eiswasser gegossen und mit Methylenchlorid ausgeschüttelt. Die organischen Phasen werden abgetrennt, mit Wasser gewaschen und über Magnesiumsulfat getrocknet. Nach Entfernung des Lösungsmittels wird das rohe 1-[1-(2-Methoxy-phenyl)-ethyl]-2-methyl-imidazol als Oel erhalten. Das Hydrochlorid schmilzt bei 201-203°.

Das als Ausgangsprodukt benötigte 1-[1-(2-Methoxyphenyl)-ethyl-2-methyl-imidazol kann wie folgt erhalten werden:

20,0 g (0,1 Mol) α-Methylen-1-(2-Hydroxybenzyl)-2-methylimidazol werden in 500 ml Ethanol gelöst und nach Zugabe von 1 g Pd-Kohle bei Normaldruck und Raumtemperatur hydriert. Anschliessend wird vom Katalysator abfiltriert, das Lösungsmittel am Vakuum weitgehend entfernt und der Rückstand mit Fumarsäure bis zur schwach sauren Reaktion versetzt. Nach Zugabe von Essigester wird das ausgefallene Produkt abfiltriert, aus Alkohol-Essigester umkristallisiert und getrocknet. Das reine 1-[1-(2-Hydroxyphenyl)-ethyl]-2-methylimidazol schmilzt bei 148-150°.

Zur weiteren Umsetzung werden 10 g (0,03 Mol) des Fumarates mit 2n Natronlauge und Ether in die freie Base gespalten. Es wird freie Base erhalten, welche wie beschrieben umgesetzt wird.

Beispiel 16: In analoger Weise wie in Beispiel 1 beschrieben, werden aus 18,2 g (0,1 Mol) 1-(2,3-Dimethoxy-phenyl)-ethanol und 25 g (0,3 Mol) 2-Methylimidazol Rohprodukt erhalten. Die freie Base wird mit Methylenchlorid/1-5 % Methanol an Silicagel chromatographiert und ergibt reines 1-[1-(2,3-Dimethoxyphenyl)-ethyl]-2-methyl-imidazol vom Smp. 122-124°. Das Hydrochlorid schmilzt bei 175-178°.

Beispiel 17: In analoger Weise wie in Beispiel 1 beschrieben, werden aus 16,4 g (0,1 Mol) 1-(2,4,6-Trimethylphenyl)-ethanol und 25 g (0,3 Mol) 2-Methylimidazol 7,9 g reines 1-[1-(2,4,6-Trimethylphenyl)-ethyl]-2-methyl-imidazol vom Smp. 129-131° erhalten. Das Hydrochlorid schmilzt bei 267° (Zers.).

Beispiel 18: In analoger Weise wie in Beispiel 1 beschrieben, werden aus 9,1 g (0,05 Mol) 1-(2,6-Dimethoxyphenyl)-ethanol und 12,5 g (0,15 Mol) 2-Methylimidazol 3,7 g rohes 1-[1-(2,6-Dimethoxyphenyl)-ethyl]-2-methyl-imidazol erhalten, welches reines Hydrochlorid vom Smp. 214-216° ergibt.

Das Ausgangsprodukt kann wie folgt hergestellt werden:

16,6 g (0,1 Mol) 2,6-Dimethoxybenzaldehyd werden in 200 ml abs. Tetrahydrofuran gelöst und 60 ml einer 2,58 molaren Methylmagnesiumjodid-Lösung in Tetrahydrofuran bei 10° zugetropft. Das Reaktionsgemisch wird 2 Stunden bei Raumtemperatur gehalten und anschliessend mit einer Lösung von 30 g Ammoniumchlorid in 200 ml Wasser versetzt. Das erhaltene Gemisch wird mit Ether ausgeschüttelt, die organischen Phasen mit Sole gewaschen und über Magnesiumsulfat getrocknet. Nach Entfernen des Lösungsmittels wird der Rückstand aus Isopropylether/Petrolether kristallisiert. Das erhaltene 1-( 2,6-Dimethoxyphenyl)-ethanol schmilzt bei 56-58°.

<u>Beispiel 19</u>: Analog wie in Beispiel 1 beschrieben, werden aus 3 g (0,02 Mol) 1-(2,3-Dimethylphenyl)-ethanol und 4,92 g (0,06 Mol) 2-Methylimidazol 1,95 g öliges 1-[1-(2,3-Dimethyl-phenyl)-ethyl]-2-methyl-imidazol erhalten. Aus 1,92 g der rohen Base wird mit etherischer Salzsäure das reine 1-[1-(2,3-Dimethylphenyl)-ethyl]-2-methyl-imidazol-hydrochlorid mit dem Smp. 244-246° hergestellt.

<u>Beispiel 20</u>: 9,25 g (0,05 Mol) 1-Phenyl-ethylbromid werden mit 20,5 g (0,25 Mol) 2-Methylimidazol in 100 ml Toluol gelöst und unter Rühren 8 Stunden auf 100° erwärmt. Das Reaktionsgemisch wird abgekühlt, mit 2n Salzsäure bis zur sauren Reaktion versetzt und die saure wässerige Phase abgetrennt. Die Toluollösung wird 3 mal mit je 50 ml 1n Salzsäure ausgeschüttelt und die vereinigten sauren, wässerigen Phasen mit konz. Natronlauge alkalisch gestellt. Das erhaltene Gemisch wird mit Ether ausgeschüttelt, die etherische Lösung über Magnesiumsulfat getrocknet und das Lösungsmittel am Vakuum entfernt. Der erhaltene Rückstand wird in Essigester gelöst und mit etherischer Salzsäure bis zur sauren Reaktion versetzt. Nach weiterer Zugabe von Ether wird das ausgefallene Produkt abfiltriert, mit Ether gewaschen und aus Cyclohexan umkristallisiert. Das reine 1-(1-Phenylethyl)-2-methyl-imidazol schmilzt bei 84-85°.

Wie in Beispiel 1 beschrieben, wird aus der Base das Hydrochlorid hergestellt. Das reine 1-(1-Phenyl-ethyl)-2-methyl-imidazohydrochlorid schmilzt bei 229-229,5°.

Beispiel 21: 23,02 g (0,11 Mol) N-(1-Phenyl-ethyl)-2-amino-acetaldehyd-dimethylacetal werden mit 10,9 g (0,1 Mol) Acetiminomethylether-hydrochlorid versetzt, in 200 ml Methanol gelöst und 4 Tage bei Raumtemperatur stehen gelassen. Das Reaktionsgemisch wird am Vakuum vom Lösungsmittel befreit, der ölige Rückstand in 250 ml Wasser gelöst und zu 500 ml konz. Salzsäure gegeben. Das Gemisch wird bei 100° zur Trockne eingedampft und der erhaltene Rückstand mit einer gesättigten Pottaschelösung alkalisch gestellt. Das erhaltene Gemisch wird 3 mal mit 500 ml Ether ausgeschüttelt, die etherischen Phasen über Magnesiumsulfat getrocknet und das Lösungsmittel am Vakuum entfernt. Der ölige Rückstand wird im Hochvakuum (6,66 Pa) destilliert, Kp. = 115-120°. Das erhaltene Produkt wird in Essigester gelöst und mit etherischer Salzsäure bis zur sauren Reaktion versetzt. Das ausgefallene Produkt wird abfiltriert und aus Isopropanol/Ether umkristallisiert. Das reine 1-(2-Phenylethyl)-2-methyl-imidazol-hydrochlorid schmilzt bei 228-229°.

Das Ausgangsmaterial kann wie folgt erhalten werden:

a) 60,1 g (0,5 Mol) Acetophenon werden in 1000 ml Methanol mit 56,78 g (0,54 Mol) 2-Aminoacetaldehyd-dimethylacetal gelöst und nach Zugabe von 10 g Palladium-Kohle bei Raumtemperatur und Normaldruck katalytisch hydriert. Nach erfolgter Wasserstoffaufnahme wird vom Katalysator abfiltriert und das Lösungsmittel am Vakuum entfernt. Der erhaltene Rückstand wird am Hochvakuum bei 60-65° (7,99 Pa) destilliert und ergibt reines N-(1-Phenyl-ethyl)-2-aminoacetaldehyddimethylacetal.

b) 82 g (2 Mol) Acetonitril werden in 65 g (2 Mol) Methanol gelöst und 500 ml 4n etherische Salzsäure zugegeben. Das Reaktionsgemisch

wird bei Raumtemperatur stehengelassen und vom ausgefallenen Produkt abfiltriert. Das erhaltene Produkt wird mit Ether gewaschen und im Vakuum bei Raumtemperatur getrocknet. Das erhaltene Acetiminomethylether-hydrochlorid schmilzt bei 97°.

Beispiel 22: 9,1 g (0,05 Mol) 1-[2-Methoxybenzyl]-imidazol werden in 90 ml Tetrahydrofuran gelöst und bei -78° 7,1 g (0,11 Mol, ent sprechend 55,4 ml 2-molarer Lösung) Butyllithium in 50 ml Tetrahydrofuran zugetropft. Das Gemisch wird 2 Stunden bei -78° gerührt und anschliessend 17,0 g (0,12 Mol) Methyljodid gelöst in 20 ml Tetrahydrofuran eingetropft. Das Reaktionsgemisch wird auf Raumtemperatur aufgewärmt und 30 Stunden bei dieser Temperatur gerührt. Anschliessend werden 50 ml Wasser langsam eingetropft und das Tetrahydrofuran am Vakuum weitgehend entfernt.

Nach Zugabe von 2n Natronlauge bis zur stark alkalischen Reaktion wird der Rückstand mit Ether ausgeschüttelt. Die organischen Phasen werden über Magnesiumsulfat getrocknet und das Lösungsmittel am Vakuum entfernt. Der erhaltene ölige Rückstand wird in Essigester gelöst und mit etherischer Salzsäure bis zur sauren Reaktion versetzt. Das ausgefallene Produkt wird abfiltriert und aus Isopropanol-Ether umkristallisiert. Das reine 1-[1-(2-Methoxy-phenyl)-ethyl]-2-methyl-imidazol-hydrochlorid schmilzt bei 201-203°.

Das Ausgangsmaterial kann wie folgt erhalten werden:

Analog wie in Beispiel 1 beschrieben. werden aus 13,8 g (0,1 Mol) 2-Methoxy-benzylalkohol und 34 g (0,5 Mol) Imidazol 9,78 g reines öliges 2-Methoxybenzylimidazol erhalten. Das erhaltene Produkt wird am Hochvakuum bei 120-130° (1,33 Pa) destilliert.

Beispiel 23: 4,0 g (20 mMol) 1-[1-(4-Aminophenyl)-ethyl]-2-methylimidazol werden in 100 ml Methanol gelöst, mit 6 ml (60 mMol) konz. Salzsäure versetzt und bei 0° 2,68 g (26 mMol) tert.-Butylnitrit eingetropft. Das Gemisch wird 30 Minuten bei 0° gerührt, anschliessend langsam im Verlaufe von 1 Stunde zum Rückfluss erhitzt und 30 Minuten bei dieser Temperatur gehalten. Das Reaktionsgemisch wird im Wasserstrahlvakuum eingeengt, mit 2n Natronlauge bis zur alkalischen Reaktion versetzt, mit Sole verdünnt und mit Ether ausgeschüttelt. Die etherischen Extrakte werden über Magnesiumsulfat getrocknet, das Lösungsmittel am Vakuum entfernt und der Rückstand mit alkoholischer Salzsäure bis zur sauren Reaktion versetzt. Nach Zugabe von Essigester wird das ausgefallene Produkt abfiltriert und getrocknet. Nach Umkristallisation aus Alkohol/Essigester wird das reine 1-[1-(4-Methoxy-phenyl)-ethyl]-2-methyl-imidazol-hydrochlorid vom Smp. 200-202° erhalten.

Das Ausgangsmaterial kann wie folgt erhalten werden:

16,5 g (0,1 Mol) p-Nitroacetophenon werden in 160 ml Methanol gelöst und langsam unter Kühlung 3,7 g (0,1 Mol) Natriumborhydrid eingetragen. Nach beendigter Zugabe wird das Gemisch langsam auf 60° erwärmt und bis zur Beendigung der Gasentwicklung bei dieser Temperatur gehalten. Nach erfolgter Abkühlung werden ca. 150 ml 1 molare Natriumdihydrogen-phosphatlösung zugegeben und das Methanol am Vakuum weitgehend entfernt. Der Rückstand wird mit Ether ausgeschüttelt, die organischen Phasen über Magnesiumsulfat getrocknet und das Lösungsmittel am Vakuum entfernt. Das erhaltene 1-[p-Nitrophenyl]-ethanol wird als rohes Oel weiterverwendet.

Aus 16 g (0,096 Mol) 1-(p-Nitrophenyl)-ethanol und 23,6 g (0,29 Mol) 2-Methylimidazol werden wie in Beispiel 1 beschrieben reines 1-[1-(4-Nitrophenyl)-ethyl]-2-methyl-imidazol vom Smp. 78-79° erhalten (Umkristallisation aus Diisopropylether-Petrolether).

14,0 g (0,06 Mol) 1-[1-(4-Nitrophenyl)-ethyl]-2-methyl-imidazol werden in 150 ml Ethanol gelöst, 2 g Raney-Nickel zugegeben und mit Wasserstoff bei Normaldruck und 20° im Verlaufe von 15 Stunden katalytisch hydriert.

Der Katalysator wird anschliessend abfiltriert, das Lösungsmittel am Vakuum entfernt und der Rückstand aus Essigester kristallisiert. Das reine 1-[1-(4-Aminophenyl)-ethyl]-2-methyl-imidazol schmilzt bei 149-151°. Das Dihydrochlorid (aus Alkohol-Essigester mit alkoholischer Salzsäure) schmilzt bei 165-167°.

Beispiel 24: In analoger Weise wie in Beispiel 23 beschrieben, kann aus 20,1 g (0,1 Mol) 1-[1-(2-Aminophenyl)-ethyl]-2-methyl-imidazol das reine 1-[1-(2-Methoxyphenyl)-ethyl]-2-methyl-imidazol-hydrochlorid vom Smp. 201-203° erhalten werden.
Das Dihydrochlorid (aus Alkohol-Essigester mit alkoholischer Salzsäure) schmilzt bei 165-167°.

Beispiel 25: Tabletten, enthaltend 0,020 g 1-(1-Phenyl-ethyl)-2-methyl-imidazol, werden z.B. wie folgt hergestellt:

| Zusammensetzung: (für 10 000 Tabletten): | |
|---|---|
| Wirkstoff | 200,00 g |
| Lactose | 290,80 g |
| Kartoffelstärke | 274,70 g |
| Stearinsäure | 10,00 g |
| Talk | 200,00 g |
| Magnesiumstearat | 2,50 g |
| Kolloidales Siliciumdioxid | 32,00 g |
| Ethanol | q.s. |

Ein Gemisch des Wirkstoffs, der Lactose und 194,70 g Kartoffelstärke wird mit einer ethanolischen Lösung der Stearinsäure befeuchtet und durch ein Sieb granuliert. Nach dem Trocknen mischt man die restliche Kartoffelstärke, den Talk, das Magnesiumstearat und das kolloidale Siliciumdioxid zu und presst die Mischung zu Tabletten von je 0,1 g Gewicht, die gewünschtenfalls mit Teilkerben zur feineren Anpassung der Dosierung versehen sein können.

Beispiel 26: Kapseln, enthaltend 0,025 g 1-(1-Phenyl-ethyl)-2-methyl-imidazol, können wie folgt hergestellt werden:

Zusammensetzung (für 1 000 Kapseln):

| | |
|---|---|
| Wirkstoff | 25,00 g |
| Lactose | 249,00 g |
| Gelatine | 2,00 g |
| Maisstärke | 10,00 g |
| Talk | 15,00 g |
| Wasser | q.s. |

Man mischt den Wirkstoff mit der Lactose, befeuchtet die Mischung gleichmässig mit einer wässerigen Lösung der Gelatine und granuliert sie durch ein Sieb mit einer Maschenweite von 1,2-1,5 mm. Das Granulat mischt man mit der getrockneten Maisstärke und dem Talk und füllt Portionen von 300 mg in Hartgelatinekapseln (Grösse 1) ab.

Patentansprüche für die Vertragsstaaten: BE, CH, LI, DE, FR, GB, IT, LU, NL, SE

1. Substituierte Imidazole der allgemeinen Formel

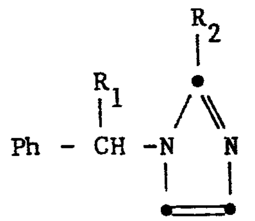

(I)

oder deren Salze, worin Ph unsubstituiertes oder durch Niederalkyl oder Niederalkoxy substituiertes Phenyl bedeutet und $R_1$ und $R_2$ jeweils Niederalkyl bedeuten.

2. Verbindungen gemäss Anspruch 1 der Formel I oder deren Salze, worin Ph unsubstituiertes oder ein- oder mehrfach durch Niederalkyl substituiertes Phenyl bedeutet und $R_1$ und $R_2$ jeweils Niederalkyl bedeuten.

3. Verbindungen gemäss Anspruch 1 der Formel I oder deren Salze, worin Ph ein- oder mehrfach durch Niederalkoxy substituiertes Phenyl bedeutet, und $R_1$ und $R_2$ jeweils Niederalkyl bedeuten.

4. Verbindungen gemäss Anspruch 1 der Formel I oder deren Salze, worin Ph unsubstituiertes oder in Position 2 oder 3 einfach durch Niederalkyl oder Niederalkoxy jeweils mit bis und mit 4 C-Atomen oder in Position 2 und 6 zweifach durch Niederalkyl mit bis und mit 4 C-Atomen substituiertes Phenyl bedeutet und $R_1$ und $R_2$ jeweils Niederalkyl mit bis und mit 4 C-Atomen bedeuten.

5. Verbindungen gemäss Anspruch 1 der Formel I oder deren Salze, worin Ph unsubstituiertes Phenyl bedeutet und $R_1$ und $R_2$ jeweils Niederalkyl mit bis und mit 4 C-Atomen bedeuten.

6. 1-(1-Phenylethyl)-2-methyl-imidazol oder ein Salz davon.

7. 1-[1-(2-Methyl-phenyl)-ethyl]-2-methyl-imidazol oder ein Salz davon.

8. 1-[1-(2-Methoxy-phenyl)-ethyl]-2-methyl-imidazol oder ein Salz davon.

9. 1-[1-(p-Methyl-phenyl)-ethyl]-2-methyl-imidazol, 1-[1-(3-Methyl-phenyl)-ethyl]-2-methylimidazol, 1-[1-(Phenyl)-propyl]-2-methyl-imidazol, 1-[1-(2-Ethyl-phenyl)-ethyl]-2-methyl-imidazol, 1-[1-(2,6-Dimethylphenyl)-ethyl]-2-methyl-imidazol, 1-[1-(3-Methoxy-phenyl)-ethyl]-2-methylimidazol, 1-[1-(2,3-Dimethoxyphenyl)-ethyl]-2-methyl-imidazol, 1-[1-(2,4,6-Trimethylphenyl)-ethyl]-2-methyl-imidazol, 1-[1-(2,6-Dimethoxyphenyl)-ethyl]-2-methyl-imidazol, 1-[1-(2,3-Dimethylphenyl)-ethyl]-2-methyl-imidazol, 1-[1-(4-Methoxy-phenyl)-ethyl]-2-methyl-imidazol oder ein Salz davon.

10. Verbindung gemäss einem der Ansprüche 1-9 in Form eines Enantiomeren.

11. Verbindung gemäss einem der Ansprüche 1-10 oder ein pharmazeutisch verwendbares Salz davon zur Anwendung in einem Verfahren zur therapeutischen oder prophylaktischen Behandlung des menschlichen oder tierischen Körpers.

12. Verbindung gemäss Anspruch 11 oder ein pharmazeutisch verwendbares Salz davon zur Behandlung von depressiven Zuständen.

13. Pharmazeutische Präparate, enthaltend eine Verbindung gemäss einem der Ansprüche 1-12 oder ein pharmazeutisch verwendbares Salz davon neben üblichen pharmazeutischen Hilfs- und Trägerstoffen.

14. Verwendung von Verbindungen gemäss einem der Ansprüche 1-12 der Formel I oder pharmazeutisch verwendbare Salze davon zur Herstellung pharmazeutischer Präparate.

15. Verfahren zur Herstellung von Verbindungen der Formel I gemäss einem der Ansprüche 1-10, dadurch gekennzeichnet, dass man

a) eine Verbindung der Formel

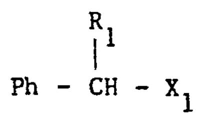

(IIa),

worin $X_1$ reaktionsfähiges verestertes Hydroxy bedeutet, mit einer Verbindung der Formel

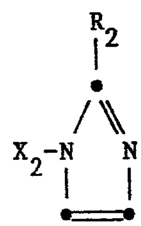

(IIb),

worin $X_2$ für Wasserstoff oder einen metallischen Rest steht, umsetzt, oder

b) in einer Verbindung der Formel

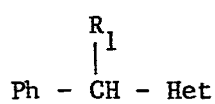

(III),

worin Het für einen in 2-$R_2$-Imidazol-1-yl überführbaren Rest steht, Het in 2-$R_2$-Imidazol-1-yl überführt, oder

c) in einer Verbindung der Formel

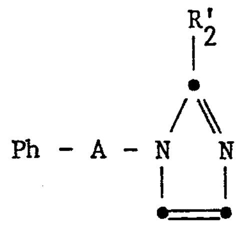

(IV),

worin A für eine in $-CH(R_1)-$ überführbare Gruppierung steht und der Rest $R'_2$ einen in $R_2$ überführbaren Rest oder $R_2$ bedeutet, oder worin A für die Gruppierung $-CH(R_1)-$ steht und $R'_2$ einen in $R_2$ überführbaren Rest bedeutet, A in $-CH(R_1)-$ und/oder $R'_2$ in $R_2$ überführt, oder

d) eine Verbindung der Formel

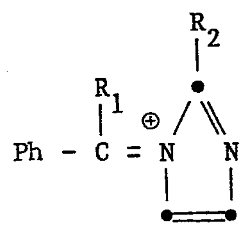

$A^{\ominus}$ (V),

worin $A^{\ominus}$ ein Anion einer Protonensäure bedeutet, zu der entsprechenden Verbindung der Formel I reduziert, oder

e) eine Verbindung der Formel

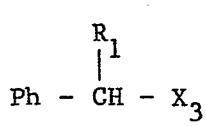

(VI),

worin $X_3$ für eine durch Cyclisierung in 2-$R_2$-Imidazol-1-yl überführbare Gruppe steht, cyclisiert, oder

f) eine Verbindung der Formel

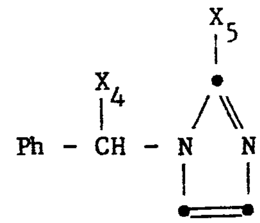

(VIIa)

mit einer Verbindung der Formel $R^o-X_6$ (VIIb) umsetzt, worin einer der Reste $X_4$ und $X_6$ reaktionsfähiges verestertes Hydroxy bedeutet und der andere für einen metallischen Rest steht, $X_5$ den Rest $R_2$ bedeutet und $R^o$ den Rest $R_1$ bedeutet, oder worin einer der Reste $X_5$ und $X_6$ reaktionsfähiges verestertes Hydroxy bedeutet und der andere für einen metallischen Rest steht, $X_4$ den Rest $R_1$ bedeutet und $R^o$ den Rest $R_2$ bedeutet, oder

g) zur Herstellung von Verbindungen der Formel I oder deren Salzen, worin Ph durch Niederalkoxy substituiertes Phenyl bedeutet, in einer Verbindung der Formel

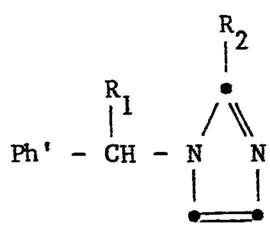

(VIII)

worin Ph' einen in Ph überführbaren Rest bedeutet, Ph' in Ph überführt,

wobei die in den Varianten a) bis g) aufgeführten Ausgangsverbindungen gegebenenfalls auch in Salzform vorliegen können, und, wenn erwünscht, eine erfindungsgemäss erhältliche Verbindung der Formel I in eine andere Verbindung der Formel I überführt und/oder gewünschtenfalls ein erhältliches Salz in die freie Verbindung der Formel I

oder in ein anderes Salz überführt und/oder gewünschtenfalls eine erfindungsgemäss erhältliche freie Verbindung der Formel I in ein Salz überführt und/oder gewünschtenfalls ein erhältliches Isomerengemisch in die einzelnen Komponenten auftrennt.

16. Verfahren zur Herstellung pharmazeutischer Präparate gemäss Anspruch 13, dadurch gekennzeichnet, dass man eine Verbindung gemäss einem der Ansprüche 1-12 oder ein pharmazeutisch verwendbares Salz davon, gegebenenfalls unter Beimischung von üblichen Hilfs- und Trägerstoffen, zu pharmazeutischen Präparaten verarbeitet.

17. Die nach dem Verfahren gemäss Anspruch 15 erhältlichen Verbindungen.

FO 7.4/DH/gs*

Patentansprüche für den Vertragsstaat AT

1. Verfahren zur Herstellung von Verbindungen der Formel

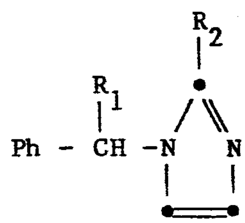

(I)

oder deren Salze, worin Ph unsubstituiertes oder durch Niederalkyl oder Niederalkoxy substituiertes Phenyl bedeutet und $R_1$ und $R_2$ jeweils Niederalkyl bedeuten, dadurch gekennzeichnet, dass man

a) eine Verbindung der Formel

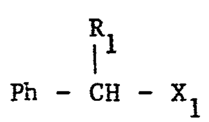

(IIa),

worin $X_1$ reaktionsfähiges verestertes Hydroxy bedeutet, mit einer Verbindung der Formel

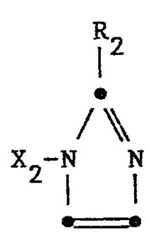

(IIb),

worin $X_2$ für Wasserstoff oder einen metallischen Rest steht, umsetzt, oder

Oesterreich

b) in einer Verbindung der Formel

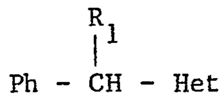 (III),

worin Het für einen in 2-$R_2$-Imidazol-1-yl überführbaren Rest steht, Het in 2-$R_2$-Imidazol-1-yl überführt, oder

c) in einer Verbindung der Formel

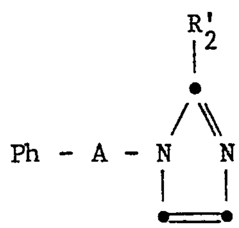 (IV),

worin A für eine in $-CH(R_1)-$ überführbare Gruppierung steht und der Rest $R_2'$ einen in $R_2$ überführbaren Rest oder $R_2$ bedeutet, oder worin A für die Gruppierung $-CH(R_1)-$ steht und $R_2'$ einen in $R_2$ überführbaren Rest bedeutet, A in $-CH(R_1)-$ und/oder $R_2'$ in $R_2$ überführt, oder

d) eine Verbindung der Formel

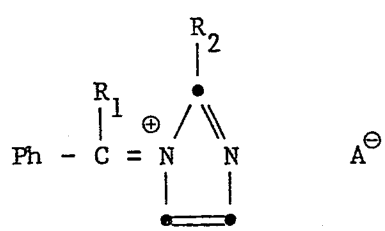 (V),

worin $A^{\ominus}$ ein Anion einer Protonensäure bedeutet, zu der entsprechenden Verbindung der Formel I reduziert, oder

e) eine Verbindung der Formel

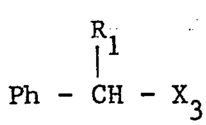 (VI),

Oesterreich

worin $X_3$ für eine durch Cyclisierung in 2-$R_2$-Imidazol-1-yl überführbare Gruppe steht, cyclisiert, oder

f) eine Verbindung der Formel

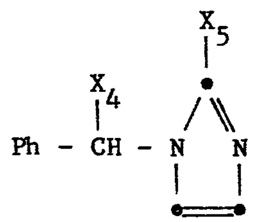

(VIIa)

mit einer Verbindung der Formel $R^o$-$X_6$ (VIIb) umsetzt, worin einer der Reste $X_4$ und $X_6$ reaktionsfähiges verestertes Hydroxy bedeutet und der andere für einen metallischen Rest steht, $X_5$ den Rest $R_2$ bedeutet und $R^o$ den Rest $R_1$ bedeutet, oder worin einer der Reste $X_5$ und $X_6$ reaktionsfähiges verestertes Hydroxy bedeutet und der andere für einen metallischen Rest steht, $X_4$ den Rest $R_1$ bedeutet und $R^o$ den Rest $R_2$ bedeutet, oder

g) zur Herstellung von Verbindungen der Formel I oder deren Salzen, worin Ph durch Niederalkoxy substituiertes Phenyl bedeutet, in einer Verbindung der Formel

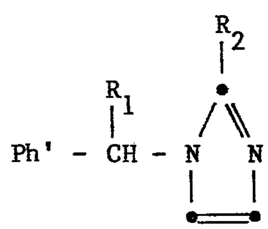

(VIII)

worin Ph' einen in Ph überführbaren Rest bedeutet, Ph' in Ph überführt,

wobei die in den Varianten a) bis g) aufgeführten Ausgangsverbindungen gegebenenfalls auch in Salzform vorliegen können, und, wenn erwünscht, eine erfindungsgemäss erhältliche Verbindung der Formel I in eine andere Verbindung der Formel I überführt und/oder gewünschtenfalls ein erhältliches Salz in die freie Verbindung der Formel I oder in ein anderes Salz überführt und/oder gewünschtenfalls eine erfindungsgemäss erhältliche freie Verbindung der Formel I in ein Salz überführt und/oder gewünschtenfalls ein erhältliches Isomerengemisch in die einzelnen Komponenten auftrennt.

2. Verfahren zur Herstellung von Verbindungen der Formel

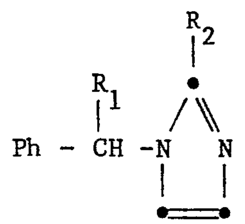

(I)

oder deren Salze, worin Ph unsubstituiertes oder durch Niederalkyl substituiertes Phenyl bedeutet und $R_1$ und $R_2$ jeweils Niederalkyl bedeuten, dadurch gekennzeichnet, dass man

a) eine Verbindung der Formel

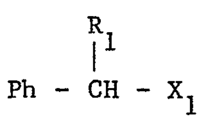

(IIa),

worin $X_1$ reaktionsfähiges verestertes Hydroxy bedeutet, mit einer Verbindung der Formel

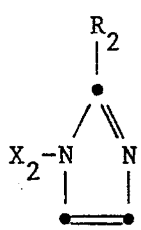

(IIb),

worin $X_2$ für Wasserstoff oder einen metallischen Rest steht, umsetzt, oder

b) in einer Verbindung der Formel

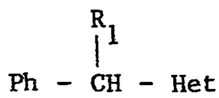 (III),

worin Het für einen in 2-$R_2$-Imidazol-1-yl überführbaren Rest steht, Het in 2-$R_2$-Imidazol-1-yl überführt, oder

c) in einer Verbindung der Formel

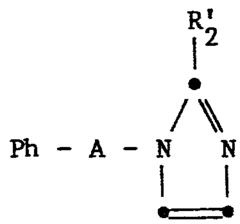 (IV),

worin A für eine in -CH($R_1$)- überführbare Gruppierung steht und der Rest $R_2'$ einen in $R_2$ überführbaren Rest oder $R_2$ bedeutet, oder worin A für die Gruppierung -CH($R_1$)- steht und $R_2'$ einen in $R_2$ überführbaren Rest bedeutet, A in -CH($R_1$)- und/oder $R_2'$ in $R_2$ überführt, oder

d) eine Verbindung der Formel

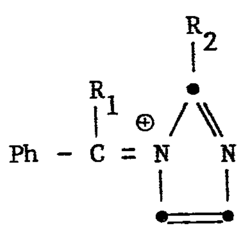 $A^{\ominus}$ (V),

worin $A^{\ominus}$ ein Anion einer Protonensäure bedeutet, zu der entsprechenden Verbindung der Formel I reduziert, oder

e) eine Verbindung der Formel

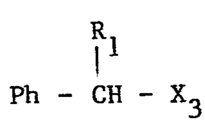 (VI),

0149976



Oesterreich

worin $X_3$ für eine durch Cyclisierung in 2-$R_2$-Imidazol-1-yl überführbare Gruppe steht, cyclisiert, oder

f) eine Verbindung der Formel

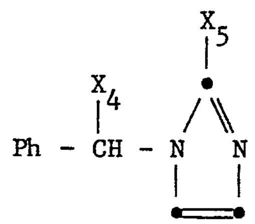

(VIIa)

mit einer Verbindung der Formel $R^o$-$X_6$ (VIIb) umsetzt, worin einer der Reste $X_4$ und $X_6$ reaktionsfähiges verestertes Hydroxy bedeutet und der andere für einen metallischen Rest steht, $X_5$ den Rest $R_2$ bedeutet und $R^o$ den Rest $R_1$ bedeutet, oder worin einer der Reste $X_5$ und $X_6$ reaktionsfähiges verestertes Hydroxy bedeutet und der andere für einen metallischen Rest steht, $X_4$ den Rest $R_1$ bedeutet und $R^o$ den Rest $R_2$ bedeutet,
wobei die in den Varianten a) bis g) aufgeführten Ausgangsverbindungen gegebenenfalls auch in Salzform vorliegen können, und, wenn erwünscht, eine erfindungsgemäss erhältliche Verbindung der Formel I in eine andere Verbindung der Formel I überführt und/oder gewünschtenfalls ein erhältliches Salz in die freie Verbindung der Formel I oder in ein anderes Salz überführt und/oder gewünschtenfalls eine erfindungsgemäss erhältliche freie Verbindung der Formel I in ein Salz überführt und/oder gewünschtenfalls ein erhältliches Isomerengemisch in die einzelnen Komponenten auftrennt.

3. Verfahren nach Anspruch 1 zur Herstellung von Verbindungen der Formel I oder deren Salze, worin Ph unsubstituiertes oder ein- oder mehrfach durch Niederalkyl substituiertes Phenyl bedeutet und $R_1$ und $R_2$ jeweils Niederalkyl bedeuten.

4. Verfahren nach Anspruch 1 zur Herstellung von Verbindungen der Formel I oder deren Salze, worin Ph ein- oder mehrfach durchch Niederalkoxy substituiertes Phenyl bedeutet, und $R_1$ und $R_2$ jeweils Niederalkyl bedeuten.

5. Verfahren nach Anspruch 1 zur Herstellung von Verbindungen der Formel I oder deren Salze, worin Ph unsubstituiertes oder in Position 2 oder 3 einfach durch Niederalkyl oder Niederalkoxy jeweils mit bis und mit 4 C-Atomen oder in Position 2 und 6 zweifach durch Niederalkyl mit bis und mit 4 C-Atomen substituiertes Phenyl bedeutet und $R_1$ und $R_2$ jeweils Niederalkyl mit bis und mit 4 C-Atomen bedeuten.

6. Verfahren nach Anspruch 1 zur Herstellung von V erbindungen der Formel I oder deren Salze, worin Ph unsubstituiertes Phenyl bedeutet und $R_1$ und $R_2$ jeweils Niederalkyl mit bis und mit 4 C-Atomen bedeuten.

7. Verfahren nach Anspruch 1 zur Herstellung von 1-(1-Phenylethyl)-2-methyl-imidazol oder einem Salz davon.

8. Verfahren nach Anspruch 1 zur Herstellung von 1-[1-(2-Methylphenyl)-ethyl]-2-methyl-imidazol oder einem Salz davon.

9. Verfahren nach Anspruch 1 zur Herstellung von 1-[1-(2-Methoxyphenyl)-ethyl]-2-methyl-imidazol oder einem Salz davon.

10. Verfahren nach Anspruch 1 zur Herstellung von 1-[1-(p-Methylphenyl)-ethyl]-2-methyl-imidazol, 1-[1-(3-Methyl-phenyl)-ethyl]-2-methylimidazol, 1-[1-(Phenyl)-propyl]-2-methyl-imidazol, 1-[1-(2-Ethyl-phenyl)-ethyl]-2-methyl-imidazol, 1-[1-(2,6-Dimethylphenyl)-ethyl]-2-methyl-imidazol, 1-[1-(3-Methoxy-phenyl)-ethyl]-2-methyl-imidazol, 1-[1-(2,3-Dimethoxyphenyl)-ethyl]-2-methyl-imidazol,

1-[1-(2,4,6-Trimethylphenyl)-ethyl]-2-methyl-imidazol, 1-[1-(2,6-Dimethoxyphenyl)-ethyl]-2-methyl-imidazol, 1-[1-(2,3-Dimethylphenyl)-ethyl]-2-methyl-imidazol, 1-[1-(4-Methoxy-phenyl)-ethyl]-2-methyl-imidazol oder einem Salz davon.

11. Verfahren nach Anspruch 1 zur Herstellung von Verbindungen gemäss einem der Ansprüche 1-10 in Form eines Enantiomeren.

12. Verfahren zur Herstellung pharmazeutischer Präparate, dadurch gekennzeichnet, dass man eine Verbindung gemäss einem der Ansprüche 1-11 oder ein pharmazeutisch verwendbares Salz davon, gegebenenfalls unter Beimischung von üblichen Hilfs- und Trägerstoffen, zu pharmazeutischen Präparaten verarbeitet.

13. Die nach dem Verfahren gemäss Anspruch 1 erhältlichen Verbindungen.

FO 7.4/DH/gs*